# EUROPEAN PATENT APPLICATION

(11) **EP 1 887 086 A1**
(43) Date of publication of application: **13.02.2008**
(21) Application number: 06746519.5
(22) Date of filing: 17.05.2006
(51) Int. Cl.: C12P 41/00

(54) **PROCESS FOR PRODUCTION OF CARBOXYLIC ACID USING SURFACTANT-MODIFYING ENZYME**

(30) Priority: 17.05.2005 JP 2005143609
(71) Applicant: Ube Industries, Ltd., Ube-shi, Yamaguchi-ken 755-8633 (JP)
(72) Inventor: MIYATA, Hiroyuki c/o Ube Research Laboratory, UBE INDUSTRIES, LTD., Yamaguchi 755-8633 (JP); KONEGAWA, Tadayoshi c/o Ube Research Laboratory, UBE INDUSTRIES, LTD., Yamaguchi 755-8633 (JP); YAMAMOTO, Yasuhito c/o Ube Research Laboratory, UBE INDUSTRIES, LTD., Yamaguchi 755-8633 (JP)
(74) Representative: HOFFMANN EITLE
(86) International application number: PCT/JP2006/309816
(87) International publication number: WO 2006/123689

(57) **Abstract**

The present invention relates to a process for preparing a carboxylic acid using a surfactant-modified enzyme which comprises selectively reacting water and a carboxylic acid ester, provided that triglyceride is excluded, in an organic solvent in the presence of a surfactant-modified enzyme.

## Description

### TECHNICAL FIELD

The present invention relates to a process for preparing a carboxylic acid by hydrolyzing a carboxylic acid ester in the presence of a surfactant-modified enzyme in a uniform solvent comprising water, and an organic solvent which forms a uniform phase with water, in particular, it relates to a process for preparing an optically active carboxylic acid (for example, an α or β-amino acid) by hydrolyzing a carboxylic acid ester (racemic mixture; for example, an α or β-amino acid ester (racemic mixture)) having an asymmetric carbon atom at other than an ester portion. Incidentally, the surfactant-modified enzyme of the present invention means a material in which an enzyme surface is covered by a surfactant, and, in view of a chemical aspect, the surface of the enzyme and a hydrophilic group(s) of the surfactant are binding with an interaction such as a hydrogen bonding, etc. The carboxylic acid, in particular, an optically active carboxylic acid (for example, an optically active α or β-amino acid) to be obtained according to the present invention is useful as a starting material or a synthetic intermediate for medicinal and agricultural chemicals, or for physiologically active substances such as physiologically active peptides or lactam series antibiotics, etc. (for example, see Patent Literatures 1-3).

### BACKGROUND ART

Heretofore, as a method for preparing a carboxylic acid by hydrolyzing a carboxylic acid ester in the presence of a surfactant-modified enzyme, it has been disclosed a method in which triglyceride is hydrolyzed in the presence of a surfactant-coated lipase in isooctane to obtain a mixture of triolein, diolein and monoolein (for example, see Non-Patent Literature 1). However, according to this method, there is no description other than hydrolysis of triglyceride.

On the other hand, as a method of preparing an optically active α or β-amino acid ester by hydrolyzing a carboxylic acid ester which is an α or β-amino acid ester (racemic mixture), it has been disclosed a method in which, for example, one of enantiomers of ethyl 3-amino-3-aryl-propionate (racemic mixture) is selectively hydrolyzed in water in the presence of a lipase (Trade name: Amano PS) originated from *Burkholderia cepacia* (*Pseudomonas cepacia)* to obtain an optically active (S)-3-amino-3-arylpropionic acid and an optically active ethyl (R)-3-amino-3-aryl-propionate (for example, see Non-Patent Literature 2.).

However, according to this method, an E value which is an index of selectivity between enantiomers by an enzyme is low, and when the produced optically active carboxylic acid is water-soluble, it is difficult to recover the product with a yielded amount of 100% from an aqueous solution after completion of the reaction. In addition, there is a problem that, in the presence of a large amount of water, optical purity is lowered due to self-hydrolysis of the substrate. Incidentally, the E value is widely utilized as an index of selectivity of kinetic optical resolution (for example, see Non-Patent Literature 3.).

Also, as a method of obtaining an optically active 3-amino-3-arylpropionic acid, it has been known a method in which good yield and good optical purity can be accomplished by making an ester portion propyl ester (for example, see Patent Literature 4).

However, according to this method, a large amount of water must be used, so that there is a problem that reaction operation is complicated, for example, a pH adjustment of an aqueous phase is indispensable, etc. Incidentally, as an optically active β-amino acid ester, a methyl ester or an ethyl ester is desired to be prepared in many cases, so that the resulting optically active β-amino acid propyl ester must be led to a desired methyl ester or an ethyl ester by interesterification, etc., whereby it is not an efficient method.

Moreover, as a method of obtaining an optically active 3-amino-3-arylpropionic acid, it has been known a method which can realize high enantio-selectivity by carrying out enzymatic hydrolysis of a 3-amino-3-arylpropionic acid ester (racemic compound) in a two-phase system comprising water and an organic solvent (for example, see Patent Literature 5).

However, according to this method, when the produced optically active carboxylic acid is water-soluble, it is difficult to recover 100% of the product from an aqueous solution after completion of the reaction, and there is a problem that optical purity of the product is lowered due to self-hydrolysis of the substrate in the presence of a large amount of water.
Non-Patent Literature 1: Chemical Engineering theses, 19(3), 424 (1993)
Non-Patent Literature 2: Tetrahedron Lett., 41, 2679(2000)
Non-Patent Literature 3: J. Am. Chem. Soc., 104, 7294(1982)
Non-Patent Literature 4: J. Org. Chem., 60, 2244(1995)
Non-Patent Literature 5: "Chemical Dictionary", published by Tokyo Kagaku Dozin Co., Ltd., p.948 (2000)
Non-Patent Literature 6: J. Member. Sci., 19, 237(1984) Patent Literature 1: WO2001/042192
Patent Literature 2: WO2004/092116
Patent Literature 3: US2003/0199692
Patent Literature 4: JP 2003-325195 A
Patent Literature 5: JP 2003-325197 A
Patent Literature 6: JP 64-80282 A
Patent Literature 7: JP 4-23981 A
Patent Literature 8: JP 6-269285 A
Patent Literature 9: JP 9-118844 A
Patent Literature' 10: JP 10-248558 A
Patent Literature 11: Japanese Patent No. 3218794

### DISCLOSURE OF THE INVENTION

### PROBLEMS TO BE SOLVED BY THE INVENTION

An object of the present invention is to solve the above-mentioned problems, and to provide a process for preparing a carboxylic acid by hydrolyzing a carboxylic acid ester with a simple and easy method, in particular, to provide a process for preparing an optically active (S or R)-α or β-substituted carboxylic acid and an optically active (R or S)-α or β-substituted carboxylic acid ester simultaneously by hydrolyzing a carboxylic acid ester (racemic mixture) having an asymmetric carbon atom at a portion other than an ester portion, for example, an α or β-substituted carboxylic acid ester (racemic mixture) with high E value.

### MEANS TO SOLVE THE PROBLEMS

The problems of the present invention can be solved by a process for preparing a carboxylic acid which comprises selectively reacting water and a carboxylic acid ester, provided that triglyceride is excluded, in an organic solvent in the presence of a surfactant-modified enzyme.

### BEST MODE FOR CARRYING OUT THE INVENTION

The carboxylic acid ester to be used in the hydrolysis of the present invention is not specifically limited, and is preferably a carboxylic acid ester (racemic mixture) having an asymmetric carbon atom at other than an ester portion, more preferably an α or β-substituted carboxylic acid ester (racemic mixture; hereinafter referred to as Compound (I)) represented by the formula (I):

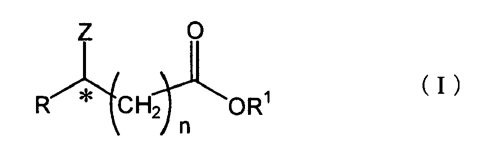

wherein R represents an alkyl group, alkenyl group, alkynyl group, cycloalkyl group, aralkyl group, aryl group or heteroaryl group, each of which may have a substituent(s), R¹ represents an alkyl group which may have a substituent(s), Z represents an amino group which may have a protective group, a hydroxyl group which may have a protective group or an alkyl group, * represents an asymmetric carbon atom, and n is 0 or 1.

R in Compound (I) represents an alkyl group, alkenyl group, alkynyl group, cycloalkyl group, aralkyl group, aryl group or heteroaryl group, each of which may have a substituent (s).

As the alkyl group of an alkyl group which may have a substituent(s) in the above-mentioned R, there may be mentioned a straight or branched alkyl group having 1 to 10 carbon atoms, for example, an alkyl group such as a methyl group, ethyl group, propyl group, butyl group, pentyl group, hexyl group, heptyl group, octyl group, nonyl group, decyl group, etc., preferably an alkyl group having 1 to 8 carbon atoms such as a methyl group, ethyl group, n-propyl group, isopropyl group, n-butyl group, n-octyl group, etc., more preferably a methyl group, or ethyl group. Incidentally, these groups contain various kinds of isomers.

As the substituent(s) of the alkyl group which may have a substituent(s), there may be mentioned, for example, a halogen atom such as a fluorine atom, chlorine atom, bromine atom, iodine atom, etc.; a hydroxyl group; an alkoxy group having 1 to 4 carbon atoms such as a methoxy group, ethoxy group, propoxy group, butoxy group, etc.; an amino group; a dialkylamino group such as a dimethylamino group, diethylamino group, etc.; a cyano group; or a nitro group, preferably a fluorine atom, chlorine atom, hydroxyl group, amino group, or dialkylamino group.

As the alkyl group having such a substituent(s), there may be mentioned, for example, a fluoromethyl group, chloromethyl group, hydroxymethyl group, methoxymethyl group, aminomethyl group, dimethylaminomethyl group, 2-chloroethyl group, 2,2-dichloroethyl group, 2-hydroxyethyl group, and 2-cyanoethyl group, etc., preferably a fluoromethyl group, chloromethyl group, hydroxymethyl group, aminomethyl group, dimethylaminomethyl group, 2-chloroethyl group, or 2-cyanoethyl group.

As the alkenyl group of an alkenyl group which may have a substituent(s) in the above-mentioned R, there may be mentioned, for example, an alkenyl group having 2 to 10 carbon atoms such as a vinyl group, propenyl group, butenyl group, pentenyl group, hexenyl group, heptenyl group, octenyl group, nonenyl group, decenyl group, etc., preferably an alkenyl group having 2 to 7 carbon atoms such as a vinyl group, propenyl group, butenyl group, pentenyl group, etc., more preferably an alkenyl group having 2 to 3 carbon atoms such as a vinyl group, 1-propenyl group, 2-propenyl group, etc. Incidentally, these groups contain various kinds of isomers.

As the substituent(s) of the alkenyl group which may have a substituent(s), there may be mentioned, for example, a halogen atom such as a fluorine atom, chlorine atom, bromine atom, iodine atom, etc.; a hydroxyl group; an alkoxyl group having 1 to 4 carbon atoms such as a methoxyl group, ethoxyl group, propoxyl group, butoxyl group, etc.; an amino group; a dialkylamino group such as a dimethylamino group, diethylamino group, etc.; a cyano group; or a nitro group, preferably a fluorine atom, chlorine atom, hydroxyl group, amino group, or dialkylamino group.

As the alkenyl group having such a substituent(s), there may be mentioned, for example, a 1-fluoroethenyl group, 1-chloroethenyl group, 1-hydroxyethenyl group, 1-methoxyethenyl group, 1-aminoethenyl group, 1-cyanoethenyl group, 2-fluoroethenyl group, 2-chloroethenyl group, 2-hydroxyethenyl group, 2-methoxyethenyl group, 2-aminoethenyl group, 2-cyanoethenyl group, 1,2-dimethylaminoethenyl group, 1-fluoro-2-propenyl group, 1-chloro-2-propenyl group, 1-hydroxy-2-propenyl group, 1-methoxy-2-propenyl group, 1-amino-2-propenyl group, 1-cyano-2-propenyl group, 3-fluoro-1-propenyl group, 3-chloro-1-propenyl group, 3-hydroxy-2-propenyl group, 3-methoxy-2-propenyl group, 3-amino-2-propenyl group, 2-cyano-2-propenyl group, 3,3-dimethylamino-2-propenyl group, 3,3-dichloro-2-propenyl group, etc., preferably 1-fluoroethenyl group, 1-chloroethenyl group, 1-hydroxyethenyl group, 1-aminoethenyl group, 1-cyanoethenyl group, 1-fluoro-2-propenyl group, 1-chloro-2-propenyl group, and 1-cyano-2-propenyl group.

As the alkynyl group of an alkynyl group which may have a substituent(s) in the above-mentioned R, there may be mentioned, for example, an alkynyl group having 2 to 10 carbon atoms such as an ethynyl group, propynyl group, butynyl group, pentynyl group, hexynyl group, heptynyl group, octynyl group, nonynyl group, decynyl group, etc., preferably an alkynyl group having 2 to 7 carbon atoms such as an ethynyl group, propynyl group, butynyl group, pentynyl group, etc., more preferably an alkynyl group having 2 to 3 carbon atoms such as an ethynyl, 1-propynyl group, 2-propynyl group, etc. Incidentally, these groups contain various kinds of isomers.

As the substituent(s) of the alkynyl group which may have a substituent(s), there may be mentioned, for example, a halogen atom such as a fluorine atom, chlorine atom, bromine atom, iodine atom, etc.; a hydroxyl group; an alkoxyl group having 1 to 4 carbon atoms such as a methoxyl group, ethoxyl group, propoxyl group, butoxyl group, etc.; an amino group; a dialkylamino group such as a dimethylamino group, diethylamino group, etc.; a cyano group; and a nitro group, preferably a fluorine atom, chlorine atom, hydroxyl group, amino group, and dialkylamino group.

As the alkynyl group having such a substituent(s), there may be mentioned, for example, a 2-fluoroethynyl group, 2-chloroethynyl group, 2-hydroxyethynyl group, 2-methoxyethynyl group, 2-aminoethynyl group, 2-cyanoethynyl group, 1-fluoro-2-propynyl group, 1-chloro-2-propynyl group, 1-hydroxy-2-propynyl group, 1-methoxy-2-propynyl group, 1-amino-2-propynyl group, 1-cyano-2-propynyl group, 1,1-dichloro-2-propynyl group, and 1,1-diamino-2-propynyl group, etc., preferably a 2-fluoroethynyl group, 2-chloroethynyl group, 2-hydroxyethynyl group, 2-aminoethynyl group, 1-fluoro-2-propynyl group, and 1,1-dichloro-2-propynyl group.

The cycloalkyl group of a cycloalkyl group which may have a substituent(s) in the above-mentioned R is a cycloalkyl group having 3 to 10 carbon atoms, and there may be mentioned, for example, a cycloalkyl group such as a cyclopropyl group, cyclobutyl group, cyclopentyl group, cyclohexyl group, cycloheptyl group, cyclooctyl group, cyclononyl group, and cyclodecyl group (Incidentally, these groups contain various kinds of isomers), preferably a cycloalkyl group having 3 to 8 carbon atoms such as a cyclopropyl group, cyclobutyl group, cyclopentyl group, cyclohexyl group, cycloheptyl group, and cyclooctyl group, etc., more preferably a cycloalkyl group having 3 to 6 carbon atoms such as a cyclopropyl group, cyclobutyl group, cyclopentyl group, and cyclohexyl group, etc.

As the substituent(s) of the cycloalkyl group which may have a substituent(s), there may be mentioned an alkyl group having 1 to 6 carbon atoms, a halogen atom such as a fluorine atom, chlorine atom, bromine atom, iodine atom, etc.; a hydroxyl group; an alkoxyl group having 1 to 4 carbon atoms such as a methoxyl group, ethoxyl group, propoxyl group, butoxyl group, etc.; an amino group; a dialkylamino group having 2 to 12 carbon atoms such as a dimethylamino group, diethylamino group, etc.; a cyano group; and a nitro group, preferably a fluorine atom, chlorine atom, hydroxyl group, amino group, and dialkylamino group.

As the cycloalkyl group which may have such a substituent(s), there may be mentioned, for example, a 1-fluorocyclopropyl group, 2-chlorocyclopropyl group, 3-fluorocyclobutyl group, methoxycyclopropyl group, aminocyclopentyl group, dimethylaminocyclohexyl group, 2-chlorocyclopropyl group, 2,2-dichlorocyclohexyl group, 2-hydroxycyclobutyl group, and 2-cyanocyclohexyl group, etc., preferably a fluorocyclopropyl group, and chlorocyclobutyl group.

The aralkyl group of an aralkyl group which may have a substituent(s) in the above-mentioned R, there may be mentioned, for example, an aralkyl group such as a benzyl group, phenethyl group, phenylpropyl group, and phenylbutyl group, etc., preferably a benzyl group, 1-phenethyl group, 2-phenethyl group, 3-phenylpropyl group, and 3-phenylbutyl group. Incidentally, these groups contain various kinds of isomers.

As the substituent(s) of the aralkyl group which may have a substituent(s), there may be mentioned, for example, an alkyl group having 1 to 10 carbon atoms such as a methyl group, ethyl group, propyl group, butyl group, pentyl group, hexyl group, heptyl group, octyl group, nonyl group, decyl group, etc. (Incidentally, these groups contain various kinds of isomers.); a hydroxyl group; a nitro group; a halogen atom such as a fluorine atom, chlorine atom, bromine atom, iodine atom, etc.; an alkoxyl group having 1 to 10 carbon atoms such as a methoxyl group, ethoxyl group, propoxyl group, butoxyl group, pentyloxyl group, hexyloxyl group, heptyloxyl group, octyloxyl group, nonyloxyl group, decyloxyl group, etc. (Incidentally, these groups contain various kinds of isomers.); an aralkyloxyl group having 7 to 10 carbon atoms such as a benzyloxyl group, phenethyloxyl group, phenylpropoxyl group, etc. (Incidentally, these groups contain various kinds of isomers.); an aryloxyl group such as a phenyloxyl group, nephthyloxyl group, etc. (Incidentally, these groups contain various kinds of isomers.); an alkoxyalkoxyl group having 2 to 12 carbon atoms such as a methoxymethoxyl group, methoxyethoxyl group, etc. (Incidentally, these groups contain various kinds of isomers.); a monoalkylamino group having 1 to 6 carbon atoms such as a methylamino group, ethylamino group, etc. (Incidentally, these groups contain various kinds of isomers.); a dialkylamino group having 2 to 12 carbon atoms such as a dimethylamino group, diethylamino group, etc. (Incidentally, these groups contain various kinds of isomers.); an acylamino group having 1 to 6 carbon atoms such as a formylamino group, acetylamino group, benzoylamino group, etc. (Incidentally, these groups contain various kinds of isomers.); a nitro group; a cyano group; a halogenated alkyl group having 1 to 6 carbon atoms such as a trifluoromethyl group, etc.

As the aralkyl group having such a substituent(s), there may be specifically mentioned, for example, a 2-fluorobenzyl group, 3-fluorobenzyl group, 4-fluorobenzyl group, 3,4-difluorobenzyl group, 2,4-difluorobenzyl group, 2-chlorobenzyl group, 3-chlorobenzyl group, 4-chlorobenzyl group, 2,4-dichlorobenzyl group, 3,4 -dichlorobenzyl group, 2-bromobenzyl group, 3-bromobenzyl group, 4-bromobenzyl group, 2,4-dibromobenzyl group, 3,4-dibromobenzyl group, 2-iodobenzyl group, 3-iodobenzyl group, 4-iodobenzyl group, 2,3-diiodobenzyl group, 3,4-diiodobenzyl group, 2-methylbenzyl group, 3-methylbenzyl group, 4-methylbenzyl group, 2-ethylbenzyl group, 3-ethylbenzyl group, 4-ethylbenzyl group, 2-hydroxybenzyl group, 3-hydroxybenzyl group, 4-hydroxybenzyl group, 2-methoxybenzyl group, 3-methoxybenzyl group, 4-methoxybenzyl group, 2,4-dimethoxybenzyl group, 3,4-dimethoxybenzyl group, 2-ethoxybenzyl group, 4-ethoxybenzyl group, 2-trifluoromethylbenzyl group, 4-trifluoromethylbenzyl group, 4-benzyloxybenzyl group, 2-nitrobenzyl group, 3-nitrobenzyl group, 4-nitrobenzyl group, 2-cyanobenzyl group, 3-cyanobenzyl group, 4-cyanobenzyl group, 4-dimethylaminobenzyl group, 4-formylaminobenzyl group, 2-acetylaminobenzyl group, 3-acetylaminobenzyl group, 4-acetylaminobenzyl group, 4-benzoylaminobenzyl group, 2-(2-fluorophenyl)ethyl group, 2-(3-fluorophenyl)ethyl group, 2-(4-fluorophenyl)ethyl group, 2-(3,4-difluorophenyl)ethyl group, 2-(2,4-difluorophenyl)ethyl group, 2-(2-chlorophenyl)ethyl group, 2-(3-chlorophenyl)ethyl group, 2-(4-chlorophenyl)ethyl group, 2-(2,4-dichlorophenyl)ethyl group, 2-(3,4-dichlorophenyl)ethyl group, 2-(2-bromophenyl)ethyl group, 2-(3-bromophenyl)ethyl group, 2-(4-bromophenyl)ethyl group, 2-(2,4-dibromophenyl)ethyl group, 2-(3,4-dibromophenyl)ethyl group, 2-(2-iodophenyl)ethyl group, 2-(3-iodophenyl)ethyl group, 2-(4-iodophenyl)ethyl group, 2-(2,3-diiodophenyl)ethyl group, 2-(3,4-diiodophenyl)ethyl group, 2-(2-tolyl)ethyl group, 2-(3-tolyl)-ethyl group, 2-(4-tolyl)ethyl group, 2-(2-ethylphenyl)ethyl group, 2-(3-ethylphenyl)ethyl group, 2-(4-ethylphenyl)ethyl group, 2-(2-hydroxyphenyl)ethyl group, 2-(4-hydroxyphenyl)-ethyl group, 2-(2-methoxyphenyl)ethyl group, 2-(3-methoxyphenyl)ethyl group, 2-(4-methoxyphenyl)ethyl group, 2-(2,4-dimethoxyphenyl)ethyl group, 2-(3,4-dimethoxyphenyl)ethyl group, 2-(2-ethoxyphenyl)ethyl group, 2-(4-ethoxyphenyl)-ethyl group, 2-(2-trifluoromethylphenyl)ethyl group, 2-(4-trifluoromethylphenyl)ethyl group, 2-(4-benzyloxyphenyl)-ethyl group, 2-(2-nitrophenyl)ethyl group, 2-(3-nitrophenyl)ethyl group, 2-(4-nitrophenyl)ethyl group, 2-(2-cyanophenyl)ethyl group, 2-(3-cyanophenyl)ethyl group, 2-(4-cyanophenyl)ethyl group, 2-(4-dimethylaminophenyl)ethyl group, 2-(4-formylaminophenyl)ethyl group, 2-(2-acetylaminophenyl)ethyl group, 2-(3-acetylaminophenyl)ethyl group, 2-(4-acetylaminophenyl)ethyl group, 2-(4-benzoylaminophenyl)ethyl group, 3-(2-fluorophenyl)propyl group, 3-(4-fluorophenyl)propyl group, 3-(4-chlorophenyl)propyl group, 3-(4-bromophenyl)propyl group, 3-(4-iodophenyl)-propyl group, 3-(2-chlorophenyl)propyl group, 3-(2-methoxyphenyl)propyl group, 3-(4-methoxyphenyl)propyl group, 3-(3,4-dimethoxyphenyl)propyl group, 3-(4-trifluoromethylphenyl)propyl group, 3-(2-trifluoromethylphenyl)propyl group, 3-(4-nitrophenyl)propyl group, 3-(4-cyanophenyl)-propyl group, 3-(4-acetylaminophenyl)propyl group, etc., preferably 2-fluorobenzyl group, 3-fluorobenzyl group, 4-fluorobenzyl group, 2-chlorobenzyl group, 3-chlorobenzyl group, 4-chlorobenzyl group, 2-bromobenzyl group, 3-bromobenzyl group, 4-bromobenzyl group, 2-iodobenzyl group, 3-iodobenzyl group, 4-iodobenzyl group, 2-methylbenzyl group, 3-methylbenzyl group, 4-methylbenzyl group, 2-hydroxybenzyl group, 4-hydroxybenzyl group, 2-methoxybenzyl group, 3-methoxybenzyl group, 4-methoxybenzyl group, 3,4-dimethoxybenzyl group, 2-trifluoromethylbenzyl group, 4-trifluoromethylbenzyl group, 4-benzyloxybenzyl group, 2-nitrobenzyl group, 3-nitrobenzyl group, 4-nitrobenzyl group, 2-cyanobenzyl group, 3-cyanobenzyl group, 4-cyanobenzyl group, 4-formylaminobenzyl group, 3-acetylaminobenzyl group, 4-acetylaminobenzyl group, 4-benzoylaminobenzyl group, 2-(2-fluorophenyl)ethyl group, 2-(3-fluorophenyl)ethyl group, 2-(4-fluorophenyl)ethyl group, 2-(2-chlorophenyl)ethyl group, 2-(3-chlorophenyl)ethyl group, 2-(4-chlorophenyl)ethyl group, 2-(2-bromophenyl) ethyl group, 2-(3-bromophenyl)ethyl group, 2-(4-bromophenyl)ethyl group, 2-(2-iodophenyl)ethyl group, 2-(3-iodophenyl)ethyl group, 2-(4-iodophenyl)ethyl group, 2-(2-tolyl)ethyl group, 2-(3-tolyl)ethyl group, 2-(4-tolyl)ethyl group, 2-(2-ethylphenyl)ethyl group, 2-(2-hydroxyphenyl)ethyl group, 2-(4-hydroxyphenyl)ethyl group, 2-(2-methoxyphenyl)ethyl group, 2-(3-methoxyphenyl)ethyl group, 2-(4-methoxyphenyl)ethyl group, 2-(2,4-dimethoxyphenyl)ethyl group, 2-(3,4-dimethoxyphenyl)ethyl group, 2-(2-trifluoromethylphenyl)ethyl group, 2-(4-trifluoromethylphenyl)ethyl group, 2-(4-benzyloxyphenyl)ethyl group, 2-(2-nitrophenyl)ethyl group, 2-(3-nitrophenyl)ethyl group, 2-(4-nitrophenyl)ethyl group, 2-(2-cyanophenyl)ethyl group, 2-(3-cyanophenyl)ethyl group, 2-(4-cyanophenyl)ethyl group, 2-(2-acetylaminophenyl)ethyl group, 2-(3-acetylaminophenyl)ethyl group, 2-(4-acetylaminophenyl)ethyl group, 2-(4-benzoylaminophenyl)ethyl group, 3-(2-fluorophenyl)propyl group, 3-(4-fluorophenyl)propyl group, 3-(4-chlorophenyl)-propyl group, 3-(4-bromophenyl)propyl group, 3-(4-iodophenyl)propyl group, 3-(2-chlorophenyl)propyl group, 3-(2-methoxyphenyl)propyl group, 3-(4-methoxyphenyl)propyl group, 3-(3,4-dimethoxyphenyl)propyl group, 3-(4-trifluoromethylphenyl)propyl group, 3-(2-trifluoromethylphenyl)-propyl group, 3-(4-nitrophenyl)propyl group, 3-(4-cyanophenyl)propyl group, 3-(4-acetylaminophenyl)propyl group, more preferably (a 2-fluorobenzyl group, 4-fluorobenzyl group, 2-chlorobenzyl group, 4-chlorobenzyl group, 2-bromobenzyl group, 4-bromobenzyl group, 2-iodobenzyl group, 4-iodobenzyl group, 2-methylbenzyl group, 4-methylbenzyl group, 4-hydroxybenzyl group, 2-methoxybenzyl group, 4-methoxybenzyl group, 3,4-dimethoxybenzyl group, 2-trifluoromethylbenzyl group, 4-trifluoromethylbenzyl group, 4-benzyloxybenzyl group, 2-nitrobenzyl group, 4-nitrobenzyl group, 2-cyanobenzyl group, 3-cyanobenzyl group, 4-cyanobenzyl group, 3-acetylaminobenzyl group, 4-acetylaminobenzyl group, 2-(2-fluorophenyl)ethyl group, 2-(4-fluorophenyl)ethyl group, 2-(2-chlorophenyl)ethyl group, 2-(4-chlorophenyl)ethyl group, 2-(2-bromophenyl)ethyl group, 2-(4-bromophenyl)ethyl group, 2-(2-iodophenyl)ethyl group, 2-(4-iodophenyl)ethyl group, 2-(2-tolyl)ethyl group, 2-(4-tolyl)ethyl group, 2-(4-hydroxyphenyl)ethyl group, 2-(2-methoxyphenyl)ethyl group, 2-(4-methoxyphenyl)ethyl group, 2-(3,4-dimethoxyphenyl)ethyl group, 2-(2-trifluoromethylphenyl)ethyl group, 2-(4-trifluoromethylphenyl)ethyl group, 2-(4-benzyloxyphenyl)ethyl group, 2-(2-nitrophenyl)ethyl group, 2-(4-nitrophenyl)ethyl group, 2-(2-cyanophenyl)ethyl group, 2-(4-cyanophenyl)ethyl group, 2-(2-acetylaminophenyl)ethyl group, and 2-(4-acetylaminophenyl)ethyl group.

As the aryl group of an aryl group which may have a substituent(s) in the above-mentioned R, there may be mentioned a phenyl group, naphthyl group, anthranyl group, phenanthryl group, biphenyl group, and binaphthyl group.

As the substituent(s) of the aryl group which may have a substituent(s), there may be mentioned an alkyl group having 1 to 4 carbon atoms such as a methyl group, ethyl group, propyl group, butyl group, etc. (Incidentally, these groups contain various kinds of isomers.); a hydroxyl group; a halogen atom such as a chlorine atom, bromine atom, iodine atom, fluorine atom, etc.; an alkoxyl group having 2 to 4 carbon atoms such as an ethoxyl group, etc. (Incidentally, these groups contain various kinds of isomers.); an alkylenedioxyl group having 1 to 4 carbon atoms such as a methylenedioxyl group, etc.; a nitro group; a cyano group; a halogenated alkyl group such as a trifluoromethyl group, etc.

As the aryl group which may have such a substituent(s), there may be mentioned, for example, a 2-tolyl group, 3-tolyl group, 4-tolyl group, 2,3-xylyl group, 2,6-xylyl group, 2,4-xylyl group, 3,4-xylyl group, mesityl group, 2-hydroxyphenyl group, 3-hydroxyphenyl group, 4-hydroxyphenyl group, 2,3-dihydroxyphenyl group, 2,4-dihydroxyphenyl group, 3,4-dihydroxyphenyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 2-iodophenyl group, 3-iodophenyl group, 4-iodophenyl group, 2-fluorophenyl group, 3-fluorophenyl group, 4-fluorophenyl group, 2,3-difluorophenyl group, 2,4-difluorophenyl group, 3,4-difluorophenyl group, 3-bromo-5-chloro-2-hydroxyphenyl group, 2-methoxyphenyl group, 3-methoxyphenyl group, 4-methoxyphenyl group, 2,3-dimethoxyphenyl group, 2,4-dimethoxyphenyl group, 3,4-dimethoxyphenyl group, 3,5-dimethoxyphenyl group, 3,4-methylenedioxyphenyl group, 4-ethoxyphenyl group, 4-butoxyphenyl group, 4-isopropoxyphenyl group, 1-phenoxyphenyl group, 4-benzyloxyphenyl group, 4-trifluoromethylphenyl group, 2-nitrophenyl group, 3-nitrophenyl group, 4-nitrophenyl group, 4-cyanophenyl group, 4-methoxycarbonylphenyl group, 1-naphthyl group, and 2-naphthyl group, etc., preferably a phenyl group, 2-tolyl group, 3-tolyl group, 4-tolyl group, 2,3-xylyl group, 2-hydroxyphenyl group, 3-hydroxyphenyl group, 4-hydroxyphenyl group, 2,3-dihydroxyphenyl group, 2,4-dihydroxyphenyl group, 3,4-dihydroxyphenyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 2-iodophenyl group, 3-iodophenyl group, 4-iodophenyl group, 2-fluorophenyl group, 3-fluorophenyl group, 4-fluorophenyl group, 3,4-difluorophenyl group, 3-bromo-5-chloro-2-hydroxyphenyl group, 2-methoxyphenyl group, 3-methoxyphenyl group, 4-methoxyphenyl group, 2,3-dimethoxyphenyl group, 2,4-dimethoxyphenyl group, 3,4-dimethoxyphenyl group, 3,5-dimethoxyphenyl group, 3,4-methylenedioxyphenyl group, 4-ethoxyphenyl group, 4-trifluoromethylphenyl group, 4-nitrophenyl group, 4-cyanophenyl group, 1-naphthyl group, and 2-naphthyl group, more preferably a phenyl group, 2-tolyl group, 3-tolyl group, 4-tolyl group, 2,3-xylyl group, 4-hydroxyphenyl group, 3,4-dihydroxyphenyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 4-iodophenyl group, 2-fluorophenyl group, 3-fluorophenyl group, 4-fluorophenyl group, 3,4-difluorophenyl group, 2-iodophenyl group, 3-iodophenyl group, 4-iodophenyl group, 2-methoxyphenyl group, 3-methoxyphenyl group, 4-methoxyphenyl group, 2,3-dimethoxyphenyl group, 2,4-dimethoxyphenyl group, 3,4-dimethoxyphenyl group, 3,5-dimethoxyphenyl group, 3,4-methylenedioxyphenyl group, 4-trifluoromethylphenyl group, 4-nitrophenyl group, 1-naphthyl group, 2-naphthyl group, 3-pyridyl group, particularly preferably phenyl group, 2-tolyl group, 3-tolyl group, 4-tolyl group, 2,3-xylyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 2-fluorophenyl group, 3-fluorophenyl group, 4-fluorophenyl group, 3,4-difluorophenyl group, 2-iodophenyl group, 3-iodophenyl group, 4-iodophenyl group, 2-methoxyphenyl group, 3-methoxyphenyl group, 4-methoxyphenyl group, 2,3-dimethoxyphenyl group, 3,4-dimethoxyphenyl group, 3,5-dimethoxyphenyl group and 3,4-methylenedioxyphenyl group.

As the heteroaryl group of a heteroaryl group which may have a substituent(s) in the above-mentioned R, there may be mentioned for example, a 2-furyl group, 3-furyl group, 2-pyridyl group, 3-pyridyl group, 4-pyridyl group, 2-pyrrolyl group, 3-pyrrolyl group, 2-thienyl group, 3-thienyl group, 2-indolyl group, 3-indolyl group, 2-imidazolyl group, 4-imidazolyl group, 3-pyrazolyl group, 2-pyrimidyl group, 4-pyrimidyl group, and quinolyl group.

As the substituent(s) of the heteroaryl group which may have a substituent(s), there may be mentioned an alkyl group having 1 to 4 carbon atoms such as a methyl group, ethyl group, propyl group, butyl group, etc. (Incidentally, these groups contain various kinds of isomers.); a hydroxyl group; a halogen atom such as a chlorine atom, bromine atom, iodine atom, fluorine atom, etc.; an alkoxyl group having 2 to 4 carbon atoms such as an ethoxyl group, etc. (Incidentally, these groups contain various kinds of isomers.); an amino group; a nitro group; a cyano group; a halogenated alkyl group such as a trifluoromethyl group, etc.

As the heteroaryl group which may have a substituent(s), there may be mentioned, for example, a 2-(3-methyl)furyl group, 2-(4-methyl)furyl group, 2-(3-ethyl)-furyl group, 2-(4-ethyl)furyl group, 2-(3-fluoro)furyl group, 2-(3-chloro)furyl group, 2-(3-hydroxy)furyl group, 2-(3-methoxy)furyl group, 2-(3-amino)furyl group, 2-(3-nitro)furyl group, 2-(3-cyano)furyl group, 2-(3-methyl)-pyridyl group, 2-(4-methyl)pyridyl group, 2-(3-ethyl)-pyridyl group, 2-(4-ethyl)pyridyl group, 2-(3-fluoro)-pyridyl group, 2-(4-chloro)pyridyl group, 2-(3-hydroxy)-pyridyl group, 2-(3-methoxy)pyridyl group, 2-(3-amino)-pyridyl group, 2-(3-nitro)pyridyl group, 2-(3-cyano)pyridyl group, 2-(3,5-dichloro)pyridyl group, 3-(2-chloro)pyridyl group, 2-(3-methyl)pyrrolyl group, and 2-(3-methyl)thienyl group, etc., preferably a 2-(3-methyl)furyl group, 2-(3-fluoro)furyl group, 2-(3-methyl)pyridyl group, 2-(3-fluoro)pyridyl group, 2-(3-nitro)pyridyl group, 2-(3-cyano)pyridyl group, and 2-(3,5-dichloro)pyridyl group.

Z in Compound (I) represents an amino group which may have a protective group, a hydroxyl group which may have a protective group or an alkyl group.

As the above-mentioned protective group, there may be mentioned, for example, a benzyl group, 4-methoxyphenylmethyl group, methoxymethyl group, trimethylsilyl group, triethylsilyl group, t-butyldimethylsilyl group, acetyl group, benzoyl group, isopropylidene acetal group, 9-fluorenylmethoxycarbonyl group, t-butoxycarbonyl group, and benzyloxycarbonyl group, etc.

As the above-mentioned alkyl group, there may be mentioned a straight or branched alkyl group having 1 to 10 carbon atoms, and there may be specifically mentioned, for example, an alkyl group such as a methyl group, ethyl group, propyl group, butyl group, pentyl group, hexyl group, heptyl group, octyl group, nonyl group, decyl group, etc. Incidentally, these groups contain various kinds of isomers.

R¹ in Compound (I) represents an alkyl group which may have a substituent(s).

As the alkyl group of the alkyl group which may have a substituent(s) in the above-mentioned R¹, there may be mentioned a straight or branched alkyl group having 1 to 10 carbon atoms, and there may be specifically mentioned, for example, an alkyl group such as a methyl group, ethyl group, propyl group, butyl group, pentyl group, hexyl group, heptyl group, octyl group, nonyl group, decyl group, etc., preferably an alkyl group having 1 to 6 carbon atoms such as a methyl group, ethyl group, n-propyl group, isopropyl group, n-butyl group, isobutyl group, sec-butyl group, n-pentyl group, n-hexyl group, etc., more preferably an alkyl group having 1 to 4 carbon atoms such as a methyl group, ethyl group, n-propyl group, n-butyl group, isobutyl group, etc. Incidentally, these groups contain various kinds of isomers.

As the substituent(s) of the alkyl group which may have a substituent(s), there may be mentioned a halogen atom such as a fluorine atom, chlorine atom, bromine atom, iodine atom, etc.; a hydroxyl group; an alkoxyl group having 1 to 4 carbon atoms such as a methoxyl group, ethoxyl group, propoxyl group, butoxyl group, etc.; a dialkylamino group such as a dimethylamino group, diethylamino group, etc.; a cyano group, etc., preferably a fluorine atom, chlorine atom, methoxyl group, ethoxyl group, hydroxyl group, and cyano group, more preferably a fluorine atom, chlorine atom, methoxyl group, and ethoxyl group.

As the alkyl group which may have such a substituent(s), there may be mentioned, for example, a 2-fluoroethyl group, 2-chloroethyl group, 2,2-difluoroethyl group, 2,2-dichloroethyl group, 2,2,2-trichloroethyl group, 2,2,2-trifluoroethyl group, 2-methoxyethyl group, 2-ethoxyethyl group, methoxymethyl group, 2-hydroxyethyl group, 2-cyanoethyl group, 2-bromoethyl group, 2-dimethylamino group, 2-chloropropyl group, 3-chloropropyl group, etc., preferably a 2-chloroethyl group, 2,2,2-trichloroethyl group, 2,2,2-trifluoroethyl group, methoxymethyl group, 2-methoxyethyl group, and 2-ethoxyethyl group.

Incidentally, n is 1 or 2.

As the surfactant-modified enzyme to be used in the hydrolysis of the present invention, for example, those enzymes in which a hydrolase is subjected to covering treatment with a surfactant and solubilized in an organic solvent are suitably used.

With regard to the above-mentioned surfactant-modified enzyme, as a hydrolase to be used as a base material, there may be mentioned, for example, a protease, esterase, lipase, etc., preferably a lipase obtained from microorganisms isolatable from yeast or bacteria, more preferably at least one kind selected from a lipase (for example, Amano PS (available from AMANO ENZYME CO.), etc.) originated from *Burkholderia cepacia* (*Pseudomonas cepacia*) and a lipase (for example, NOVOZYM 435 (available from NOVOZYMS COM.), etc.) originated from *Candida Antarctica* is used. Incidentally, as these hydrolases, commercially available product can be used as such as a natural form or an immobilized enzyme, and may by used alone or in admixture of two or more kinds. Also, an enzyme-immobilizing agent contained in the commercially available product is previously removed and then the treated product may be used.

The above-mentioned hydrolases are desirably used after subjecting the commercially available product sold as a natural form or an immobilized enzyme to chemical treatment or physical treatment.

The synthetic method of the surfactant-modified enzyme having the above-mentioned characteristics is not specifically limited, and it may be a preparation method in which the product shows properties wherein it has high solubility in an organic solvent, and the dissolved enzyme fraction shows high activity in an organic solvent. There may be mentioned a method in which, for example, a surfactant dissolved in a small amount of methanol, ethanol, propanol, acetone, methyl ethyl ketone, or other hydrophilic organic solvents is mixed under cooling with a buffer in which a hydrolase had been dissolved therein, to precipitate a surfactant-modified enzyme. This precipitate is separated by ultracentrifugation or filtration, etc., washed with a buffer and distilled water, and lyophilized as such to obtain a powder state surfactant-modified enzyme (for example, see the above-mentioned Patent Literatures 6 to 10 and Non-Patent Literature 4). Or else, a surfactant dissolved in a small amount of methanol, ethanol, propanol, acetone, methyl ethyl ketone, or other hydrophilic organic solvents is mixed under cooling with a buffer in which a hydrolase had been dissolved therein, and the resulting mixed solution is lyophilized to obtain a powder state surfactant-modified enzyme (described in the following Reference example 2). Also, it may be obtained by vigorously stirring an aqueous hydrolase solution and a water-insoluble organic solvent in the presence of a surfactant which can form a reverse phase emulsion to form an emulsion and then drying the same (for example, see the above-mentioned Patent Literature 11).

Incidentally, lyophilization is a method in which an aqueous solution and a substance containing water are frozen rapidly at a temperature not more than the freezing point, and a pressure is reduced to the water vapor pressure or lower of the frozen product to remove water by sublimation whereby drying the substance (for example, see Non-Patent literature 5).

As the above-mentioned buffer, there may be mentioned, for example, an aqueous solution of an inorganic acid salt such as an aqueous sodium phosphate solution, an aqueous potassium phosphate solution, etc.; an aqueous solution of an organic acid salt such as an aqueous sodium acetate solution, an aqueous ammonium acetate solution, an aqueous sodium citrate solution, etc., preferably an aqueous sodium phosphate solution, an aqueous potassium phosphate solution, an aqueous ammonium acetate solution is/are used. Incidentally, these buffers may be used singly or in admixture of two or more kinds.

A concentration of the above-mentioned buffer is preferably 0.005 to 2 mol/L, more preferably 0.01 to 0.5 mol/L, and a pH of the buffer is preferably 4 to 9, more preferably 7 to 8.5.

An amount of the buffer to be used at the time of lyophilization is not particularly limited so long as it is a concentration that the surfactant-modified enzyme is completely dissolved, and preferably 10 ml to 1000 ml, more preferably 10 ml to 100 ml based on 1 g of the hydrolase.

As the hydrolase to be used for the preparation of the above-mentioned surfactant-modified enzyme, a commercially available product which is available as a natural form or an immobilized enzyme may be used as such.

An amount of the above-mentioned hydrolase to be used is preferably 0.1 to 1000 mg, more preferably 1 to 200 mg based on 1 g of Compound (I).

As the above-mentioned surfactant, a surfactant having both of a hydrophobic group such as a long-chain alkyl group, and a hydrophilic group is suitably used, and it may be either a natural surfactant or a synthetic surfactant. Depending on a kind of the hydrophilic group, the surfactant is classified into four groups such as an anionic surfactant, a cationic surfactant, a nonionic surfactant, and an amphoteric surfactant. Incidentally, these surfactants may be used alone or in admixture of two or more kinds.

As the above-mentioned anionic surfactant, there may be mentioned, for example, soap, a base material for soap, metal soap, triethanol amine N-acyl-L-glutamate, sodium N-acyl-L-glutamate, sodium alkyl sulfate, polyoxyethylene alkyl ether sodium sulfate, polyoxyethylene alkyl ether phosphate, polyoxyethylene alkylphenyl ether phosphate, sodium palm oil fatty acid methyl taurine, lauryl sulfate triethanol amine, lauryl sodium lauryl sulfate, sodium lauroyl sarcosinate, sodium lauroylmethyl α-alanine solution or sodium lauroylmethyl taurine, etc.

As the above-mentioned cationic surfactant, there may be mentioned, for example, invert soap, ethyl sulfate lanolin fatty acid aminopropylethyl dimethyl ammonium, alkyltrimethyl ammonium chloride, dialkyldimethyl ammonium chloride, distearyldimethyl ammonium chloride, stearyl dimethylbenzyl ammonium chloride, benzalkonium chloride or benzethonium chloride, etc.

As the above-mentioned nonionic surfactant, there may be mentioned, for example, polyethylene glycol, sorbitan alkyl ester, polyoxyethylene glycol alkyl ester, polyoxyethylene glycol alkyl ether, dialkyl glutamate gluconamide, dimethicone copolyol, sucrose aliphatic ester, sucrose stearic ester, polyoxyethylene sorbit tetraoleate, poly(oxyethylene·oxypropylene)methylpolysiloxane copolymer, polyoxyethylene octylphenyl ether, polyoxyethylene hardened castor oil, polyoxyethylene stearyl ether, polyoxyethylene stearyl amide, polyoxyethylene cetyl ether, polyoxyethylene polyoxypropyleneglycol, polyglycerin aliphatic acid ester, polyoxyethylene sorbitan monooleate, ethylene glycol monostearate, glycerin monostearate, sorbitan monostearate, propylene glycol monostearate, polyoxyethylene glycerin monostearate, polyoxyethylene sorbitan monostearate, polyoxyethylene sorbitan monopalmitate, polyethylene glycol monolaurate, polyoxyethylene sorbitan monolaurate, polyoxyethylene sorbit monolaurate or diethanol amide laurate, etc.

As the above-mentioned amphoteric surfactant, there may be mentioned, for example, 2-alkyl-N-carboxymethyl-N-hydroxyethyl imidazolium betaine, alkyldiaminoethyl glycine chloride solution or lauryl dimethylamino acetic acid betaine, etc.

As the synthetic example of the above-mentioned synthetic surfactant, it can be easily synthesize by, for example, refluxing L-glutamic acid, and a long chain alcohol such as dodecanol, oleyl alcohol, etc. in toluene in the presence of p-toluenesulfonic acid to prepare L-glutamic acid di-long chain ester, and further, refluxing the resulting ester product with δ-gluconolactone in methanol (for example, see the above-mentioned Non-Patent Literature 6).

As the above-mentioned synthetic surfactant, N-D-glucono-L-glutamic acid diesters are most suitably used.

The hydrolysis of the present invention is usually carried out in a uniform phase solvent containing water, and an organic solvent which forms a uniform phase with water. Incidentally, there is substantially no bad effect in the hydrolysis of the present invention even when water is not completely dissolved in the organic solvent to form a suspended state.

As the above-mentioned water, purified water such as deionized water and distilled water, etc. is usually used, and water may contain an inorganic salt such as sodium phosphate, potassium phosphate, etc., or an organic salt such as sodium acetate, ammonium acetate, sodium citrate, etc.

An amount of the above-mentioned water to be used is not specifically limited so long as the reaction solution forms a uniform phase, and preferably 0.5 to 10 mol, more preferably 0.5 to 5.0 mol, particularly preferably 1.0 to 3.0 mol based on 1 mol of Compound (I).

As the above-mentioned organic solvent, there may be mentioned, for example, an aliphatic hydrocarbon such as n-pentane, n-hexane, n-heptane, n-octane, cyclopentane, cyclohexane, cyclopentane, etc.; an aromatic hydrocarbon such as benzene, toluene, xylene, etc.; an ether such as diethyl ether, t-butyl methyl ether, diisopropyl ether, cyclopentyl methyl ether, tetrahydrofuran, 1,4-dioxane, etc.; a ketone such as acetone, methyl ethyl ketone, etc.; and an ester such as ethyl acetate, butyl acetate, etc., preferably n-hexane, n-heptane, cyclopentane, cyclohexane, toluene, diisopropyl ether, t-butyl methyl ether, cyclopentyl methyl ether, tetrahydrofuran, more preferably n-hexane, cyclohexane, toluene, diisopropyl ether, t-butyl methyl ether, cyclopentyl methyl ether, particularly preferably cyclohexane, toluene, or t-butyl methyl ether is used. Incidentally, these organic solvents may be used alone or in admixture of two or more kinds.

An amount of the above-mentioned organic solvent to be used is preferably 2 to 200 mL, more preferably 5 to 80 mL based on 1 g of Compound (I).

The hydrolysis of the present invention can be carried out by a method, for example, where Compound (I), a surfactant-modified enzyme, water (if necessary, it may contain an inorganic salt or an organic salt) and an organic solvent which can form a uniform phase with water are mixed and reacted under stirring, etc. A reaction temperature at that time is preferably 0 to 80°C, more preferably 10 to 50°C, particularly preferably 30 to 45°C, and a reaction pressure is not particularly limited.

In the hydrolysis of the present invention, when an α or β-substituted carboxylic acid ester (racemic mixture) represented by the formula (I):

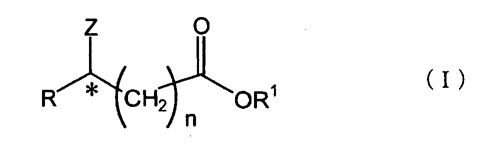

wherein R, R¹, Z, n and * have the same meanings as defined above,
is used, an optically active α or β-substituted carboxylic acid (hereinafter referred to as Compound (II)) represented by the formula (II):

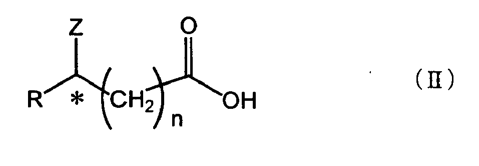

wherein R, Z, n and * have the same meanings as defined above,
and an optically active α or β-substituted carboxylic acid ester (hereinafter referred to as Compound (III)) represented by the formula (III):

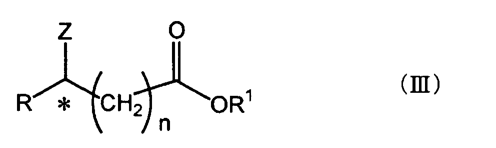

wherein R, R¹, Z, n and * have the same meanings as defined above,
which is remained and not hydrolyzed can be obtained simultaneously.

Compound (II) and Compound (III) obtained by the hydrolysis of the present invention can be separated by, for example, after completion of the reaction, filtering the precipitated Compound (II) as such, or adding a suitable organic solvent (for example, acetonitrile, acetone, etc.) to the reaction mixture and filtering to obtain Compound (II), and concentrating the organic layer after adjusting a pH of the filtrate to obtain Compound (III). Also, sodium chloride may be added to the mixture before separation to improve the recovery rate of Compound (II). Incidentally, the resulting Compound (II) and Compound (III) may be further purified according to the usual method such as crystallization, recrystallization, distillation, column chromatography, etc.

### EXAMPLES

Next, the present invention is explained more specifically by referring to Examples, but the scope of the present invention is not limited by these.

### Reference example 1 (Preparation of surfactant-modified enzyme A)

To 50 mL of 0.1 mol/L phosphate buffer (a phosphate buffer prepared by adding 0.1 mol/L aqueous disodium hydrogen phosphate solution to 0.1 mol/L aqueous sodium dihydrogen phosphate solution and adjusted to pH 7.0) was added 200 mg of a lipase (Amano Lipase PS (trade name); available from Aldrich) originated from *Burkholderia cepacia* (*Pseudomonas cepacia*)*,* and the mixture was stirred at room temperature. After 30 minutes, insoluble materials were removed by ultracentrifugation, and cooled to 4°C. To the obtained enzyme solution was added dropwise a solution in which 400 mg of didodecyl N-D-glucono-L-glutamate was dissolved in 2.5 mL of ethanol, and the mixture was stirred at 4°C. After 4 hours, stirring was stopped and the mixture was allowed to stand for 58 hours. The supernatant was removed by ultracentrifugation, and the remaining precipitate was washed twice with 0.1 mol/L phosphate buffer (pH 7.0), and once with distilled water. The solid after washing was lyophilized to obtain 180 mg of a surfactant-modified enzyme A as white powder.

### Reference example 2 (Preparation of surfactant-modified enzyme B)

To 200 mL of 0.01 mol/L phosphate buffer (a phosphate buffer prepared by adding 0.01 mol/L aqueous disodium hydrogen phosphate solution to 0.01 mol/L aqueous sodium dihydrogen phosphate solution and adjusted to pH 7.0) was added 1.60 g of a lipase (Amano Lipase PS (trade name); available from Aldrich) originated from *Burkholderia cepacia* (*Pseudomonas cepacia*)*,* and the mixture was stirred at room temperature. After 30 minutes, insoluble materials were removed by ultracentrifugation, and cooled to 4°C. To the obtained enzyme solution was added dropwise a solution in which 1.60 g of didodecyl N-D-glucono-L-glutamate was dissolved in 20.0 mL of ethanol, and the mixture was stirred at 4°C. After 4 hours, stirring was stopped and the mixture was allowed to stand for 66 hours. The mixture was lyophilized to obtain 1.87 g of a surfactant-modified enzyme B as white powder.

### Reference example 3 (Synthesis of 3-amino-3-phenylpropionic acid (racemic mixture))

To 250 mL of isopropyl alcohol were added 17.7 g (0.17 mol) of benzaldehyde, 18.2 g (0.17 mol) of malonic acid and 25.6 g (0.33 mol) of ammonium acetate, and the materials were reacted under reflux (80 to 90°C) for 7 hours while stirring. After completion of the reaction, the resulting reaction solution was stirred at 0 to 5°C for 1 hour and filtered to obtain 19.2 g (Isolation yield based on benzaldehyde: 70.0%) of 3-amino-3-phenylpropionic acid (racemic mixture) as white powder.
Incidentally, physical properties of the 3-amino-3-phenylpropionic acid (racemic mixture) are as follows.

¹H-NMR (δ (ppm), D₂O+DCl): 3.06 (dd, 1H, J=17.1, 6.8Hz), 3.17 (dd, 1H, J=17.1, 7.3Hz), 4.76 (dd, 1H, J=7.3, 6.8Hz), 3.77 (s, 2H), 7.45 (m, 5H)
¹³C-NMR (δ (ppm), D₂O+DCl): 40.5, 54.4, 130.0, 132.3, 132.6, 138.0, 176.3
MS (EI) m/z: 165 (M⁺)
MS (CI, i-C₄H₁₀) m/z: 166 (MH⁺)

| | | | | |
|---|---|---|---|---|
| Elemental analysis; | Calcd: | C, 65.44%; | H, 6.71%; | N, 8.48% |
| | Found: | C, 65.18%; | H, 6.78%; | N, 8.34% |

### Reference example 4 (Synthesis of ethyl 3-amino-3-phenylpropionate (racemic mixture))

To 6.00 mL (103 mmol) of ethanol were added 2.00 g (12.1 mmol) of 3-amino-3-phenylpropionic acid (racemic mixture) synthesized in Reference example 3 and 1.78 g (18.2 mmol) of conc. sulfuric acid, and the materials were reacted under stirring at 60°C for 4 hours. After completion of the reaction, the obtained reaction mixture was concentrated under reduced pressure, 6 mol/L aqueous sodium hydroxide solution was added to the residue, and a pH of the reaction mixture was adjusted to 8.5. Then, the mixture was extracted with 10 mL of ethyl acetate and 4 mL of water, and the organic layer was dried over anhydrous magnesium sulfate. After filtration, the filtrate was concentrated under reduced pressure to obtain 1.98 g (Isolation yield based on 3-amino-3-phenylpropionic acid (racemic mixture): 84.5%) of 3-amino-3-phenylpropionic acid ethyl ester (racemic mixture) as colorless liquid.
Incidentally, physical properties of the 3-amino-3-phenylpropionic acid ethyl ester (racemic mixture) are as follows.

¹H-NMR (δ (ppm), CDCl₃): 1.19 (t, 3H, J=7.3Hz), 3.15 (dd, 1H, J=7.3, 16.6Hz), 3.25 (dd, 1H, J=7.3, 16.6Hz), 4.15 (q, 2H, J=7.3Hz), 4.85 (dd, 1H, J=7.3, 7.3Hz), 7.50-7.55 (m, 5H)
¹³C-NMR (δ (ppm), CDCl₃): 16.0, 40.9, 54.3, 65.2, 129.9, 132.2, 132.5, 137.8, 174.3
MS (EI) m/z: 193 (M⁺)
MS (CI, i-C₄H₁₀) m/z: 194 (MH⁺)

### Example 1 (Synthesis of (S)-3-amino-3-phenylpropionic acid and ethyl (R)-3-amino-3-phenylpropionate)

To 1.0 mL of t-butyl methyl ether saturated with water were added 100 mg (0.517 mmol) of ethyl 3-amino-3-phenylpropionate (racemic mixture) and 10.0 mg of the surfactant-modified enzyme A prepared in Reference example 1, and the materials were reacted under stirring at 30°C. After 46 hours, 0.5 mL of acetone was added to the reaction mixture and the mixture was filtered to obtain 34.2 mg (Isolation yield based on ethyl 3-amino-3-phenylpropionate (racemic mixture)=40.0%) of (S)-3-amino-3-phenylpropionic acid.
The (S)-3-amino-3-phenylpropionic acid was led to n-propyl (S)-3-amino-3-phenylpropionate according to the conventional manner, and an optical purity thereof was measured by using high performance liquid chromatography which uses an optically active column, then it was 99.9%ee.
The ethyl (R)-3-amino-3-phenylpropionate was led to ethyl (R)-3-(2-furoylamino)-3-phenylpropionate according to the conventional manner, and an optical purity thereof was measured by using high performance liquid chromatography which uses an optically active column, then it was 96.6%ee.
Incidentally, the E value in the present reaction was 5522.

### Analytical conditions of high performance liquid chromatography;

Optically active n-propyl 3-amino-3-phenylpropionate Column: CHIRAL CD-Ph (0.46 cmΦx25 cm, available from SHISEIDO CO., LTD.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperature: 30°C
Wavelength: 220 nm

### Optically active ethyl 3-(2-furoylamino)-3-phenylpropionate Column: CHIRAL CEL OJ-H (0.46cmΦx 25cm, available from DAICEL CHEMICAL INDUSTRIES, LTD.)

Solvent: hexane/isopropyl alcohol (=9/1 (volume ratio))
Flow rate: 0.5 mL/min
Temperature: 30°C
Wavelength: 220 nm

Also, physical properties of the (S)-3-amino-3-phenylpropionic acid were the same as those shown in Reference example 3.
Physical properties of the ethyl (R)-3-amino-3-phenylpropionate were the same as those shown in Reference example 4.

### Example 2 (Syntheses of (S)-3-amino-3-phenylpropionic acid and ethyl (R)-3-amino-3-phenylpropionate)

To 1.0 mL of t-butyl methyl ether saturated with water were added 100 mg (0.517 mmol) of ethyl 3-amino-3-phenylpropionate (racemic mixture) and 5.0 mg of the surfactant-modified enzyme B prepared in Reference example 2, and the materials were reacted under stirring at 30°C. After 90 hours, 0.5 mL of acetone was added to the reaction mixture and the mixture was filtered to obtain 34.4 mg (Isolation yield based on ethyl 3-amino-3-phenylpropionate (racemic mixture)=40.2%) of (S)-3-amino-3-phenylpropionic acid.
The (S)-3-amino-3-phenylpropionic acid was led to n-propyl (S)-3-amino-3-phenylpropionate according to the conventional manner, and an optical purity thereof was measured by using high performance liquid chromatography which uses an optically active column, then it was 99.9%ee.
The ethyl (R)-3-amino-3-phenylpropionate was led to ethyl (R)-3-(2-furoylamino)-3-phenylpropionate according to the conventional manner, and an optical purity thereof was measured by using high performance liquid chromatography which uses an optically active column, then it was 96.3%ee.
Incidentally, the E value in the present reaction was 8106.

### Analytical conditions of high performance liquid chromatography;

### Optically active n-propyl 3-amino-3-phenylpropionate Column: CHIRAL CD-Ph (0.46 cmΦx25 cm, available from SHISEIDO CO., LTD.)

Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperature: 30°C
Wavelength: 220 nm

### Optically active ethyl 3-(2-furoylamino)-3-phenylpropionate Column: CHIRAL CEL OJ-H (0.46 cmΦx 25cm, available from DAICEL CHEMICAL INDUSTRIES, LTD.)

Solvent: hexane/isopropyl alcohol (=9/1 (volume ratio))
Flow rate: 0.5 mL/min
Temperature: 30°C
Wavelength: 220 nm

Also, physical properties of the (S)-3-amino-3-phenylpropionic acid were the same as those shown in Reference example 3.
Physical properties of the ethyl (R)-3-amino-3-phenylpropionate were the same as those shown in Reference example 4.

### Reference example 5 (Synthesis of ethyl 2-amino-4-phenylbutyrate (racemic mixture))

To 6.00 mL (103 mmol) of ethanol were added 2.00 g (11.2 mmol) of 2-amino-4-phenylbutyric acid (racemic mixture) and 1.78 g (16.8 mmol) of conc. sulfuric acid, and the materials were reacted under stirring at 60°C for 4 hours. After completion of the reaction, the obtained reaction mixture was concentrated under reduced pressure, 6 mol/L aqueous sodium hydroxide solution was added to the residue, and a pH of the mixture was adjusted to 8.5.
Then, the mixture was extracted with 10 mL of ethyl acetate and 4 mL of water, and the organic layer was dried over anhydrous magnesium sulfate. After filtration, the filtrate was concentrated under reduced pressure to obtain 1.89 g (Isolation yield based on 2-amino-4-phenylbutyric acid (racemic mixture): 82.0%) of ethyl 2-amino-4-phenylbutyrate (racemic mixture) as colorless liquid.
Incidentally, physical properties of the ethyl 2-amino-4-phenyl butyrate (racemic mixture) are as follows.

¹H-NMR (δ (ppm), CDCl₃): 1.28 (t, 3H, J=7.3Hz), 1.81-1.90 (m, 2H), 2.06 (ddd, 1H, J=5.4, 6.4, 13.7Hz), 2.08 (ddd, 1H, J=5.4, 6.4, 13.7Hz), 3.44 (dd, 1H, J=5.4, 7.8Hz), 4.18 (q, 2H, J=7.3Hz), 7.17-7.30 (m, 5H)
¹³C -NMR (δ (ppm), CDCl₃): 14.3, 32.0, 36.5, 54.1, 60.9, 126.1, 128.4, 128.5, 141.4, 176.0
MS (EI) m/z: 207 (M⁺)
MS (CI, i-C₄H₁₀) m/z: 208 (MH⁺)

### Example 3 (Syntheses of (S)-2-amino-4-phenylbutyric acid and ethyl (R)-2-amino-4-phenylbutyrate)

To 1.0 mL of t-butyl methyl ether saturated with water were added 100 mg (0.482 mmol) of ethyl 2-amino-4-phenylbutyrate (racemic mixture) and 10.0 mg of the surfactant-modified enzyme A prepared in Reference example 1, and the materials were reacted under stirring at 30°C. After 66 hours, 0.5 mL of acetone was added to the reaction mixture and the mixture was filtered to obtain 35.5 mg (Isolation yield based on ethyl 2-amino-4-phenylbutyrate (racemic mixture)=41.0%) of (S)-2-amino-4-phenylbutyric acid.
The (S)-2-amino-4-phenylbutyric acid was led to ethyl (S)-2-amino-4-phenylbutyrate according to the conventional manner, and an optical purity thereof was measured by using high performance liquid chromatography which uses an optically active column, then it was 61.5%ee.
The ethyl (R)-2-amino-4-phenybutyrate was led to ethyl (R)-2-(2-furoylamino)-4-phenylbutyrate according to the conventional manner, and an optical purity thereof was measured by using high performance liquid chromatography which uses an optically active column, then it was 93.5%ee.
Incidentally, the E value in the present reaction was 14.

### Analytical conditions of high performance liquid chromatography;

Optically active ethyl 2-amino-4-phenylbutyrate
Column: CHIRAL CD-Ph (0.46 cmΦx25 cm, available from
SHISEIDO CO., LTD.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperature: 30°C
Wavelength: 220 nm

### Optically active ethyl 2-(2-furoylamino)-4-phenylbutyrate Column: CHIRAL CEL OJ-H (0.46cmΦx 25cm, available from DAICEL CHEMICAL INDUSTRIES, LTD.)

Solvent: hexane/isopropyl alcohol (=9/1(volume ratio)) Flow rate: 0.5 mL/min
Temperature: 30°C
Wavelength: 220 nm

Also, physical properties of the (S)-2-amino-4-phenylbutyric acid are as follows.
¹H-NMR (δ (ppm), CDCl₃): 1.97-2.19 (m, 2H), 2.54-2.69 (m, 2H), 3.91 (t, 1H, J=6.4Hz), 7.09-7.22 (m, 5H)
¹³C-NMR (δ (ppm), CDCl₃): 33.2, 34.3, 55.3, 129.6, 131.4, 131.8, 143.0, 174.6
MS (EI) m/z: 179 (M⁺)
MS (CI, i-C₄H₁₀) m/z: 180 (MH⁺)

Physical properties of the ethyl (R)-2-amino-4-phenybutyrate were the same as those shown in Reference example 5.

### Example 4 (Syntheses of (S)-2-amino-4-phenylbutyric acid and ethyl (R)-2-amino-4-phenybutyrate)

To 1.0 mL of t-butyl methyl ether saturated with water were added 100 mg (0.482 mmol) of ethyl 2-amino-4-phenylbutyrate (racemic mixture) and 10.0 mg of the surfactant-modified enzyme B prepared in Reference example 2, and the materials were reacted under stirring at 30°C. After 70 hours, 0.5 mL of acetone was added to the reaction mixture and the mixture was filtered to obtain 34.9 mg (Isolation yield based on ethyl 2-amino-4-phenylbutyrate (racemic mixture)=40.3%) of (S)-2-amino-4-phenylbutyric acid.
The (S)-2-amino-4-phenylbutyric acid was led to ethyl (S)-2-amino-4-phenylbutyrate according to the conventional manner, and an optical purity thereof was measured by using high performance liquid chromatography which uses an optically active column, then it was 75.1%ee.
The ethyl (R)-2-amino-4-phenybutyrate was led to ethyl (R)-2-(2-furoylamino)-4-phenylbutyrate according to the conventional manner, and an optical purity thereof was measured by using high performance liquid chromatography which uses an optically active column, then it was 84.6%ee.
Incidentally, the E value in the present reaction was 19.

### Analytical conditions of high performance liquid chromatography;

Optically active ethyl 2-amino-4-phenylbutyrate
Column: CHIRAL CD-Ph (0.46 cmΦx25 cm, available from
SHISEIDO CO., LTD.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperature: 30°C
Wavelength: 220 nm

### Optically active ethyl 2-(2-furoylamino)-4-phenylbutyrate Column: CHIRAL CEL OJ-H (0.46 cmΦx25 cm, available from DAICEL CHEMICAL INDUSTRIES, LTD.)

Solvent: hexane/isopropyl alcohol (=9/1 (volume ratio)) Flow rate: 0.5 mL/min
Temperature: 30°C
Wavelength: 220 nm

Also, physical properties of the (S)-2-amino-4-phenylbutyric acid were the same as those shown in Example 3.
Physical properties of the ethyl (R)-2-amino-4-phenylbutyrate were the same as those shown in Reference example 5.

### Reference example 6 (Synthesis of ethyl 2-amino-3-phenylpropionate (racemic mixture))

To 10.0 mL (171 mmol) of ethanol were added 2.00 g (12.1 mmol) of 2-amino 3-phenylpropionic acid (racemic mixture) and 1.42 g (14.5 mmol) of conc. sulfuric acid, and the materials were reacted under stirring at 60°C for 4 hours. After completion of the reaction, the obtained reaction mixture was concentrated under reduced pressure, 6 mol/L aqueous sodium hydroxide solution was added to the mixture, and a pH of the reaction mixture was adjusted to 8.5. Then, the mixture was extracted with 10 mL of t-butyl methyl ether and 4 mL of water, and the organic layer was dried over anhydrous magnesium sulfate. After filtration, the filtrate was concentrated under reduced pressure to obtain 2.34 g (Isolation yield based on 2-amino 3-phenylpropionic acid (racemic mixture): 89.0%) of ethyl 2-amino 3-phenylpropionate (racemic mixture) as colorless liquid.
Incidentally, physical properties of the ethyl 2-amino 3-phenylpropionate (racemic mixture) are as follows.

¹H-NMR (δ (ppm), CDCl₃): 1.22 (t, 3H, J=7.1Hz), 2.85 (dd, 1H, J=7.8, 13.5Hz), 3.06 (dd, 1H, J=5.4, 13.5Hz), 3.69 (dd, 1H, J=5.4, 7.8Hz), 7.17-7.30 (m, 5H)
¹³C-NMR (δ (ppm), CDCl₃): 14.2, 41.2, 55.9, 60.8, 126.7, 128.5, 129.3.137.4, 175.0
MS (CI, i-C₄H₁₀) m/z: 194 (MH⁺)

| | | | | |
|---|---|---|---|---|
| Elemental analysis; | Calcd: | C, 68.37%; | H, 7.82%; | N, 7.25% |
| | Found: | C, 66.29%; | H, 7.69%; | N, 7.02% |

### Example 5 (Syntheses of (S)-2-amino-3-phenylpropionic acid and ethyl (R)-2-amino-3-phenylpropionate)

To 1.00 mL of t-butyl methyl ether saturated with water were added 100 mg (0.517 mmol) of ethyl 2-amino 3-phenylpropionate (racemic mixture) and 5.0 mg of the surfactant-modified enzyme A prepared in Reference example 2, and the materials were reacted under stirring at 30°C. After 24 hours, 0.5 mL of acetone was added to the reaction mixture and the mixture was filtered to obtain 35.8 mg (Isolation yield based on 2-amino-3-phenylpropionic acid ethyl ester (racemic mixture)=41.8%) of (S)-2-amino-3-phenylpropionic acid and a lipase as a mixture.
The (S)-2-amino-3-phenylpropionic acid was led to ethyl (S)-2-(2-furoylamino)-3-phenylpropionate according to the conventional manner, and an optical purity thereof was measured by using high performance liquid chromatography which uses an optically active column, then it was 95.9%ee.
The ethyl (R)-2-amino-3-phenylpropionate was led to ethyl (R)-2-(2-furoylamino)-3-phenylpropionate according to the conventional manner, and an optical purity thereof was measured by using high performance liquid chromatography which uses an optically active column, then it was 91.7%ee.
Incidentally, the E value in the present reaction was 155.

### Analytical conditions of high performance liquid chromatography;

### Optically active ethyl 2-(2-furoylamino)-3-phenylpropionate Column: CHIRAL CEL OJ-H (0.46 cmΦx25 cm, available from DAICEL CHEMICAL INDUSTRIES, LTD.)

Solvent: hexane/isopropyl alcohol (=8/2(volume ratio))
Flow rate: 0.5 mL/min
Temperature: 30°C
Wavelength: 220 nm

Also, physical properties of the (S)-2-amino-3-phenylpropionic acid are as follows.

¹H-NMR (δ (ppm), CD₃OD) : 3.12 (dd, 1H, J=8.0, 14.5Hz), 3.29 (dd, 1H, J=5.2, 14.5Hz), 3.99 (dd, 1H, J=5.2, 8.OHz), 7.32-7.45 (m, 5H)
¹³C-NMR (δ (ppm), CD₃OD): 39.2, 58.9, 130.5, 132.0, 132.2, 138.0, 176.8
MS (CI, i-C₄H₁₀) m/z: 166 (MH⁺)

| | | | | |
|---|---|---|---|---|
| Elemental analysis; | Calcd: | C, 65.44%; | H, 6.71%; | N, 8.48% |
| | Found: | C, 61.36%; | H, 6.69%; | N, 7.94% |

Physical properties of the ethyl (R)-2-amino 3-phenylpropionate were the same as those shown in Reference example 1.

### UTILIZABILITY IN INDUSTRY

The present invention relates to a process for preparing a carboxylic acid by hydrolyzing a carboxylic acid ester in a uniform solvent comprising water and an organic solvent which can form a uniform phase with water, in the presence of a surfactant-modified enzyme, in particular, it relates to a process for preparing an optically active carboxylic acid (for example, α or β-amino acid) by hydrolyzing a carboxylic acid ester (racemic mixture; for example, α or β-amino acid ester (racemic mixture)) having an asymmetric carbon atom at a portion other than an ester portion. The carboxylic acid obtainable by the present invention, in particular, the optically active carboxylic acid (for example, optically active α or β-amino acid) is useful as a starting material or a synthetic intermediate for medicinal and agricultural chemicals, or for physiologically active substances such as physiologically active peptide or lactam series antibiotics, etc.

## Claims

1. A process for preparing a carboxylic acid using a surfactant-modified enzyme which comprises selectively reacting water and a carboxylic acid ester, provided that triglyceride is excluded, in an organic solvent in the presence of a surfactant-modified enzyme.

2. The preparation process according to Claim 1, wherein the carboxylic acid ester is a carboxylic acid ester which is a racemic mixture and having an asymmetric carbon atom at the portion other than an ester portion.

3. The preparation process according to Claim 2, wherein the carboxylic acid ester which is a racemic mixture and having an asymmetric carbon atom at the portion other than an ester portion is an α or β-substituted carboxylic acid ester which is a racemic mixture and represented by the formula (I): 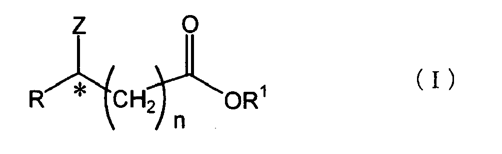 wherein R represents an alkyl group, alkenyl group, alkynyl group, cycloalkyl group, aralkyl group, aryl group or heteroaryl group, each of which may have a substituent(s), R¹ represents an alkyl group which may have a substituent(s), Z represents an amino group which may have a protective group, a hydroxyl group which may have a protective group or an alkyl group, * represents an asymmetric carbon atom, and n is 0 or 1,
and the carboxylic acid is an optically active α or β-substituted carboxylic acid represented by the formula (II) : 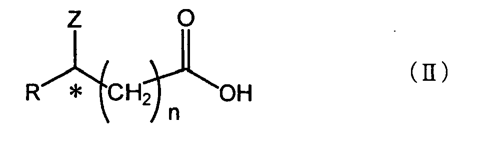 wherein R, Z, n and * have the same meanings as defined above.

4. The preparation process according to Claim 2 or 3, wherein the carboxylic acid ester (racemic mixture) having an asymmetric carbon atom at the portion other than an ester portion is an α or β-substituted carboxylic acid ester represented by the formula (I): 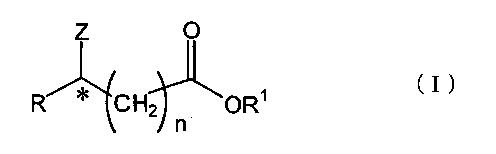 wherein R, R¹, Z, n and * have the same meanings as defined above,
the optically active carboxylic acid is an optically active α or β-substituted carboxylic acid represented by the formula (II): 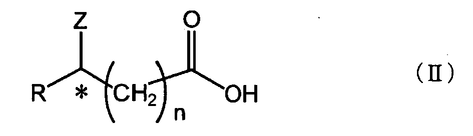 wherein R, Z, n and * have the same meanings as defined above,
and the carboxylic acid ester which is not hydrolyzed and remained is an optically active α or β-substituted carboxylic acid ester represented by the formula (III): 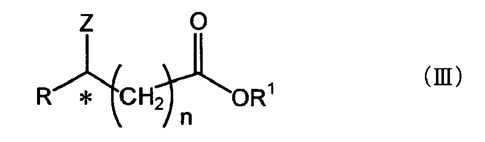 wherein R, R¹, Z, n and * have the same meanings as defined above,
provided that it has a reverse absolute configuration to that of the compound of the formula (II).

5. The preparation process according to Claim 1, wherein the surfactant-modified enzyme is an enzyme in which a hydrolase is subjected to coating treatment with a surfactant to be solubilized in an organic solvent.

6. The preparation process according to Claim 1 or 5, wherein the hydrolase is a protease, an esterase or a lipase.

7. The preparation process according to Claim 6, wherein the hydrolase is at least one hydrolase selected from the group consisting of a lipase originated from *Burkholderia cepacia* (*Pseudomonas cepacia*) and a lipase originated from *Candida Antarctica.*

8. The preparation process according to Claim 5, wherein the surfactant is N-D-glucono-L-glutamic acid diester.

9. The preparation process according to Claim 1, wherein an amount of water to be used is 0.5 to 10 mol based on 1 mol of the carboxylic acid ester.

10. The preparation process according to Claim 1, wherein the organic solvent is at least one organic solvent selected from the group consisting of an ether, a ketone, an ester, an aliphatic hydrocarbon and an aromatic hydrocarbon.

11. The preparation process according to Claim 3 or 4, wherein R¹ is a methyl group or an ethyl group each of which may have a substituent(s).

12. The preparation process according to Claim 11, wherein the substituent(s) of R¹ is a halogen atom or an alkoxy group.

13. The preparation process according to Claim 3 or 4, wherein Z is an amino group substituted by an amino group or an aralkyl group.

14. The preparation process according to Claim 4, wherein each of the optically active α or β-substituted carboxylic acid represented by the formula (II): 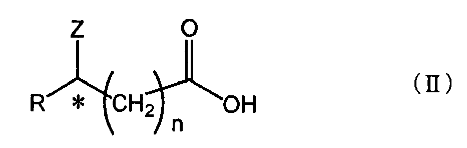 wherein R, Z, n and * have the same meanings as defined above,
which had formed by the hydrolysis, and the remaining α or β-substituted carboxylic acid ester represented by the formula (III): 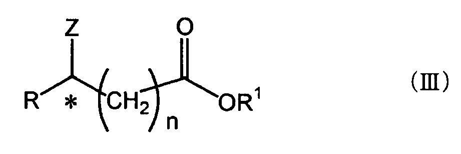 wherein R, R¹, Z, n and * have the same meanings as defined above,
which had not been hydrolyzed, provided that it has a reverse absolute configuration to that of the compound of the formula (II),
is isolated from a mixture thereof.
